# EUROPEAN PATENT APPLICATION

(11) **EP 4 524 977 A1**
(43) Date of publication of application: **19.03.2025**
(21) Application number: 23802454.1
(22) Date of filing: 24.02.2023
(51) Int. Cl.: G16H 15/00

(54) **MEDICAL REPORT GENERATION METHOD AND APPARATUS**

(30) Priority: 07.05.2022 CN 202210489271; 07.05.2022 CN 202210489219
(71) Applicant: Danyang Huichuang Medical Equipment Co., Ltd., Zhenjiang, Jiangsu 212300 (CN)
(72) Inventor: WANG, Daifa, Zhenjiang, Jiangsu 212300 (CN); DENG, Hao, Zhenjiang, Jiangsu 212300 (CN)
(74) Representative: Petraz, Gilberto Luigi
(86) International application number: PCT/CN2023/078116
(87) International publication number: WO 2023/216678

(57) **Abstract**

Provided in the present disclosure are a medical report generation method and an apparatus. The medical report generation method comprises: presenting an analysis display interface based on medical data, so that at least one display area, which is used for displaying a corresponding analysis result, is distributed on the analysis display interface; receiving an extraction operation by a user on the analysis display interface; in response to the extraction operation, generating content blocks respectively corresponding to each analysis result and prominently presenting the same on the analysis display interface, wherein the content blocks are arranged in one-to-one correspondence with the display areas on the analysis display interface; receiving a selection operation by the user on at least one content block; and generating a medical report based on the at least one content block selected by the user. The medical report generation method has high universality in processing medical data, especially in the application of processing near-infrared data, simplifies operations while ensuring the doctors' degree of freedom of selection, and reduces the information load during the doctor's operation, thereby facilitating the doctor's operation and use.

## Description

### TECHNICAL FIELD

The present disclosure relates to a field of medical technology, and in particular to a medical report generation method, an apparatus, and a functional near-infrared spectroscopy system.

### BACKGROUND

In current common medical systems, after an initial or subsequent examination is performed on a subject (usually a patient), a medical report containing information such as test information and diagnosis information is generated based on collected data. The medical report may be used for doctors' diagnosis and treatment, medical information exchange, etc. However, for medical report software in the prior art, display areas, number of displayed images, arrangement manners, etc., are fixed by default, so that doctors do not have the freedom to select and adjust, and the arrangement manners cannot be automatically adjusted according to the generated results. Therefore, the medical report software does not have universality.

Especially in the field of functional near-infrared spectroscopy, the functional near-infrared spectroscopy technology is applicable to diagnostic analysis of various different diseases, such as depression, cognitive impairment and stroke rehabilitation evaluation. As analysis and diagnosis indices of each disease are different, the display of results for doctors to analyze and diagnose is also different, for example, whether the analysis and diagnosis results are displayed in images or tables may be different as desired by the individual doctor, the displayed images and tables may vary in number. However, as required by the prior functional near-infrared spectroscopy apparatus, the doctors need to access manually screenshot software on the apparatus and intercept an analysis result display interface using the screenshot software by themselves, so as to obtain the content they want to put in the medical report. The operation is cumbersome, and the content intercepted by themselves is prone to contain redundant information. Therefore, there is a need to provide a medical report generation method that is simple to operate, convenient for doctors to use, and applicable to the analysis and diagnosis of different diseases.

### SUMMARY

In view of the above technical problems existing in the prior art, the present disclosure provides a medical report generation method and an apparatus. The medical report generation method has high universality, simplifies the operations while ensuring the doctors' degree of freedom of selection, and reduces the information load during the doctor's operation, thereby facilitating the operation and use by the doctor.

In a first aspect, an embodiment of the present disclosure provides a medical report generation method. The medical report generation method may include the following steps. An analysis display interface based on medical data may be presented, so that at least one display area, which is used for displaying a corresponding analysis result, is distributed on the analysis display interface. The medical report generation method may include receiving a first extraction operation by a user with respect to the analysis display interface. The medical report generation method may include, in response to the first extraction operation, generating content blocks corresponding to the respective analysis results and presenting prominently the same on the analysis display interface. Wherein, the content blocks are provided in one-to-one correspondence with the display areas on the analysis display interface. The medical report generation method may include receiving a selection operation by the user with respect to at least one content block, and generating a medical report based on the at least one content block selected by the user.

In a second aspect, an embodiment of the present disclosure further provides a medical report generation apparatus. The medical report generation apparatus may at least include a processor, wherein the processor is configured to perform the medical report generation method of any embodiment of the present disclosure. The medical report generation method may include presenting an analysis display interface based on medical data, so that at least one display area, which is used for displaying a corresponding analysis result, is distributed on the analysis display interface. The medical report generation method may include receiving a first extraction operation by a user with respect to the analysis display interface. The medical report generation method may include, in response to the first extraction operation, generating content blocks corresponding to the respective analysis results and presenting prominently the same on the analysis display interface. Wherein the content blocks are provided in one-to-one correspondence with the display areas on the analysis display interface. The medical report generation method may include receiving a selection operation by the user with respect to at least one content block. And the medical report generation method may include generating a medical report based on the at least one content block selected by the user.

In a third aspect, an embodiment of the present disclosure further provides a storage medium storing a computer program, wherein the computer program, when executed by a processor, implements the medical report generation method of any embodiment of the present disclosure. The medical report generation method may include presenting an analysis display interface based on medical data, so that at least one display area, which is used for displaying a corresponding analysis result, is distributed on the analysis display interface. The medical report generation method may include receiving a first extraction operation by a user with respect to the analysis display interface. The medical report generation method may include, in response to the first extraction operation, generating content blocks corresponding to the respective analysis results and presenting prominently the same on the analysis display interface. Wherein the content blocks are provided in one-to-one correspondence with the display areas on the analysis display interface. The medical report generation method may include receiving a selection operation by the user with respect to at least one content block, and generating a medical report based on the at least one content block selected by the user.

In a fourth aspect, an embodiment of the present disclosure further provides a functional near-infrared spectroscopy system. The functional near-infrared spectroscopy system at least includes the medical report generation apparatus of any embodiment of the present disclosure.

Compared with the prior art, the embodiments of the present disclosure have the following beneficial effects. In the embodiments of the present disclosure, after the first extraction operation by the user with respect to the analysis display interface is received, the content block can be presented prominently in an automatic manner. Further, the content block includes a corresponding analysis result, and a medical report may be generated according to the selected content block to reflect the analysis result as desired. In this way, a doctor can directly obtain a required medical report based on the selected content block through simple operations, without the need for manual repeating the operations. The medical report generation method has high universality in the application of functional near-infrared spectroscopy field, reduces the difficulty in generating medical reports, simplifies operations while ensuring the doctors' degree of freedom of selection, and reduces the information load during the doctor's operation, thereby facilitating the doctor's operation and use.

### BRIEF DESCRIPTION OF THE DRAWINGS

In figures that are not necessarily drawn to scale, the same reference numerals may describe similar components in different figures. The same reference signs with suffixes or different suffixes may denote different examples of similar components. The figures generally show various embodiments by way of example rather than limitation, and are used together with the description and the claims to describe the embodiments of the present disclosure. As proper, the same reference sign may be used throughout the drawings to denote the same or similar part. Such embodiments are illustrative, and are not intended to be exhaustive or exclusive embodiments of the present device, apparatus or method.
FIG. 1 is a flowchart of a medical report generation method according to an embodiment of the present disclosure.
FIG. 2 is an example diagram of an analysis display interface for auxiliary analysis of a cerebral injury condition according to a first aspect of the present disclosure.
FIG. 3 is an example diagram of an analysis display interface according to a second aspect of the present disclosure.
FIG. 4 is a partial enlarged view of a central node atlas in FIG. 3.
FIG. 5 is an example diagram of a report editing interface according to a first embodiment of the present disclosure.
FIG. 6 is an example diagram of a report editing interface according to a second embodiment of the present disclosure.
FIG. 7 is an example diagram of a report editing interface according to a third embodiment of the present disclosure.
FIG. 8 is an example diagram of a report editing interface according to fourth embodiment of the present disclosure.
FIG. 9 is an example diagram of a report editing interface according to fifth embodiment of the present disclosure.
FIG. 10 is an example diagram of a report editing interface according to a sixth embodiment of the present disclosure.
FIG. 11 is an example diagram of a picture library (gallery) interface according to an embodiment of the present disclosure.
FIG. 12 is an example diagram of a report editing interface according to a seventh embodiment of the present disclosure.
FIG. 13 is an example diagram of an analysis display interface according to a third aspect of the present disclosure.
FIG. 14 is an example diagram of a template interface according to an embodiment of the present disclosure.
FIG. 15 is an example diagram of a report editing interface according to an eighth embodiment of the present disclosure.
FIG. 16 is a structural block diagram of a functional near-infrared spectroscopy system according to an embodiment of the present disclosure.

The components indicated by the reference signs in the figures are as follows:
100-functional near-infrared spectroscopy system; 101-medical report generation apparatus; 110-processor; 1-content block; 2-box to be filled; 3-image arrangement module; 4-reference information; 5- filling block.

### DETAILED DESCRIPTION

Various solutions and features of the present application are described herein with reference to the accompanying drawings.

It should be understood that various modifications can be made to embodiments/aspects of the present disclosure herein. Therefore, the above description should not be regarded as a limitation, but only as an example of an embodiment. Those skilled in the art will conceive of other modifications within the scope and spirit of the present application.

The accompanying drawings, which are included in and constitute a part of the description, show embodiments of the present application, and are used to explain the principles of this disclosure together with the general description of this disclosure given above and the detailed description of the embodiments given below.

These and other features of the present application will become apparent from the following description of preferred forms of embodiments given as non-limiting examples with reference to the accompanying drawings.

It should also be understood that although the present application has been described with reference to some specific examples, those skilled in the art can definitely implement many other equivalent forms of the present application.

When combined with the drawings, the above and other aspects, features and advantages of the present application will become more apparent in view of the following detailed description.

Hereafter, specific embodiments of the present application will be described with reference to the accompanying drawings. However, it should be understood that the embodiments are only examples of the present application, which can be implemented in a variety of manners. Familiar and/or repetitive functions and structures are not described in detail to avoid unnecessary or superfluous details that obscure the present application. Therefore, the specific structural and functional details of the invention herein are not intended to be limited, but are only used as the basis and representative basis of the claims to teach those skilled in the art to use this application in a diversified manner with any virtually suitable detailed structure.

The description may use the phrases "in one embodiment", "in another embodiment", "in yet another embodiment" or "in other (some) embodiments", which may refer to one or more of the same or different embodiments according to this application.

An embodiment of the present disclosure provides a medical report generation method. The medical report generation method is applicable to a medical report generation apparatus, a medical report generation platform, etc., including but not limited to the field of functional near-infrared spectroscopy.

The embodiments shown in the present disclosure are applicable to the field of functional near-infrared spectroscopy, and are also applicable to other fields such as computed tomography imaging (CT imaging), magnetic resonance imaging (MRI), functional magnetic resonance imaging (fMRI), ultrasound imaging and other medical imaging fields, and are also applicable to fields such as diagnostic analysis based on physiological signals, as long as physiological information and/or biochemical information of a subject can be obtained, and a medical analysis result can be generated by means of a computer (or processor) based on the physiological information and/or biochemical information, which will not be specifically limited in the present disclosure.

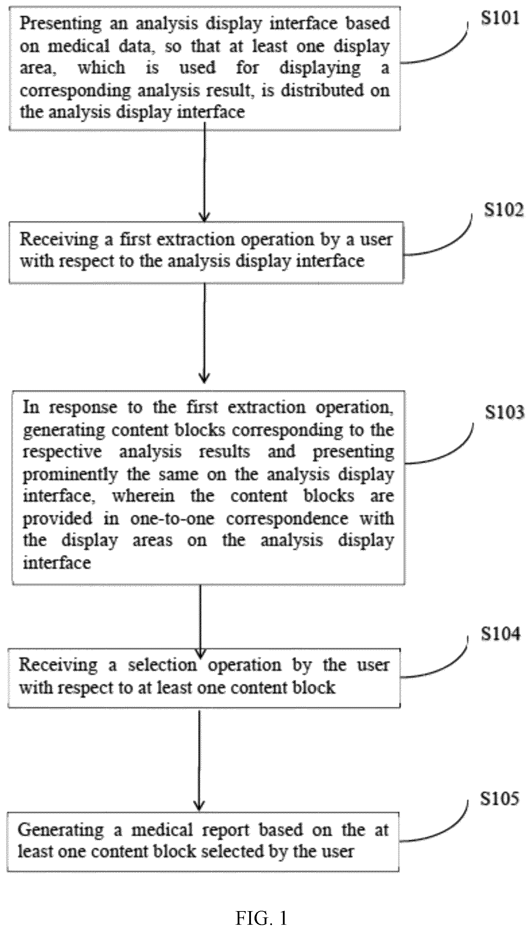

Further, as shown in FIG. 1, a medical report generation method includes steps S101 to S105.

At step S101, an analysis display interface based on medical data is presented, so that at least one display area, which is used for displaying a corresponding analysis result, is distributed on the analysis display interface.

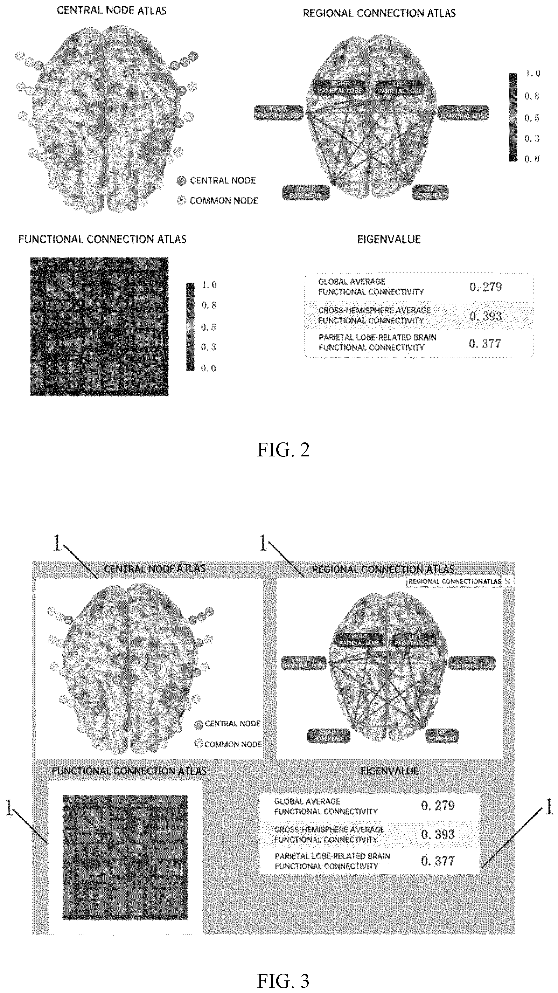

It is understandable that, the analysis result corresponding to the medical data may be a direct presentation of the medical data, for example, may be physiological and biochemical test data displayed in the form of images, tables, etc., or may be an analysis result obtained by analyzing the medical data. For example, a central node atlas shown in FIG. 2 is obtained by performing functional brain network analysis on near-infrared data of a brain of the subject. In some embodiments of the present disclosure, a category to which the medical data belongs, a manner for obtaining the medical analysis result, and a method for presenting the medical analysis result are not specifically limited.

FIG. 2 shows an example diagram of a display interface for auxiliary analysis on a cerebral injury condition, as an example, the present disclosure uses the near-infrared data as medical data for illustration. The above near-infrared data is near-infrared data of the brain of the subject, which is not limited thereto in the present disclosure. The interface shown in FIG. 2 is an analysis display interface based on the near-infrared data, and the analysis display interface may display a central node atlas, a regional connection atlas, a functional connection atlas and an eigenvalue table. Besides, the analysis display interface may also display other analysis results such as a graph, profile, and/or a table based on the near-infrared data that are used to represent the brain function of the subject.

It should be noted that the technical solution of the present disclosure is described below by taking a medical report generation method for auxiliary analysis of a cerebral injury condition as an example, but the present disclosure is not limited thereto. The analysis display interface may be an analysis display interface of any disease, such as depression, cognitive impairment, stroke rehabilitation evaluation, or the like.

It may be understood that the near-infrared data may be any data obtained based on an functional near-infrared spectroscopy apparatus, and characteristic parameters such as HBO (oxyhemoglobin), HbR (deoxyhemoglobin) and HbT (total hemoglobin) may be obtained based on the near-infrared data. The analysis results may be understood as the presentation of the cause of the disease, the pathogenesis, the changes in the structure, function and metabolism of cells, tissues and organs that occur during the disease development, and the results generated by the rules. The presentation form may specifically include text, images, tables and other forms.

It may be understood that the analysis results corresponding to the near-infrared data may be a direct presentation based on the near-infrared data, for example, may be an HBO graph (profile), or may be analysis results obtained by analyzing the near-infrared data. In summary, the analysis results may be directly obtained based on the near-infrared data, without requiring the user to edit the substantive content of the results. For example, the central node atlas shown in FIG. 2 is obtained by performing functional brain network analysis on the near-infrared data of the brain of the subject. The category to which the near-infrared data belongs, the manner for obtaining the analysis result, the method for presenting the analysis result, and the like are not specifically limited in the present disclosure.

In some optional embodiments, the display areas on the analysis display interface may be one or more, the display areas may be in one-to-one correspondence with different analysis results, and as the appearance characteristics such as size and color presented by the analysis results differ, the appearance characteristics such as size and color corresponding to the display area may also be different.

At step S102, a first extraction operation by a user with respect to the analysis display interface is received.

It should be noted that the user herein may include a doctor, a subject, or other people who need to view the medical report.

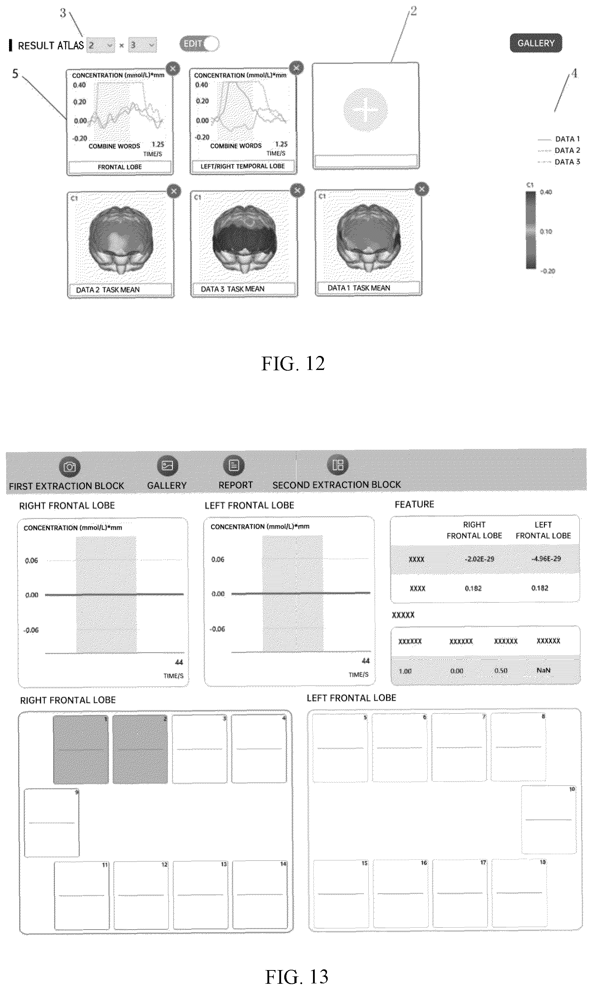

In some optional embodiments, an extraction module corresponding to the first extraction operation may be presented on the analysis display interface. For example, the "first extraction block" shown in FIG. 13 may be understood as an extraction module corresponding to the first extraction operation. When the user selects the extraction module by using an input device such as a mouse, the first extraction operation may be generated accordingly. In some other embodiments, the first extraction operation may also be generated by means of operations such as triggering a shortcut key, etc. The manner for generating the first extraction operation is not limited in the present disclosure, as long as it facilitates the user to generate the first extraction operation.

At step S103, in response to the first extraction operation, content blocks 1 corresponding to the respective analysis results are generated and presented prominently on the analysis display interface, wherein the content blocks 1 are provided in one-to-one correspondence with the display areas on the analysis display interface.

In some optional embodiments, the above first extraction operation may be understood as extracting data information contained in an analysis result corresponding to a display area, and generating a prominently presented content block 1 on the analysis display interface upon extracting the data information. The content contained in the content block 1 is the above analysis result, and the specific presentation form of the content block 1 can be text, images, tables, and other forms. For example, as shown in FIG. 2 and FIG. 3, after the first extraction operation is received, the analysis display interface shown in FIG. 2 will automatically present the prominently presented content blocks 1 as shown in FIG. 3, and the content blocks 1 are provided in one-to-one correspondence with the display areas.

In some optional embodiments, the above first extraction operation may also be understood as extracting an analysis result corresponding to a display area, for example, extracting a central node atlas, and generating a prominently presented content block 1 on the analysis display interface after extracting the central node atlas.

In some optional embodiments, the content block 1 may include one or more of a central node atlas, a regional connection atlas, a functional connection atlas, an eigenvalue table or a distribution graph (profile). The eigenvalue table may be related to a functional connection value between brain regions, and the distribution graph may be used to compare and display a representative HBO distribution graph, a representative HbR distribution graph and a representative HbT distribution graph on the same time axis. In some embodiments, the content blocks 1 may be automatically "presented" in various manners in response to the first extraction operation, so that each content block 1 is presented prominently as compared with the background portion, and thus the user may view intuitively the block-shaped content block 1, thereby facilitating reduction of information interference and the information processing load of the user. As shown in FIG. 3, a block area where the content block 1 is located and the background portion may be presented in different colors. However, this is merely an example, and the content block 1 may be "presented" prominently in various forms such as a block diagram and a mask, which are not repeated here.

For example, as shown in FIG. 3, four content blocks 1 are presented on the analysis display interface, and the number of the content blocks 1 are arranged in one-to-one correspondence with the number of the display areas on the analysis display interface, that is, as shown in FIG. 2, a number of display areas shown in FIG. 2 is four, and after the first extraction operation is received, four content blocks 1 (as shown in FIG. 3) corresponding to the number of the display areas will be generated automatically, without manual extraction by a user. The number of display areas and the number of the content blocks 1 presented on the analysis display interface are not specifically limited in the present disclosure.

In some optional embodiments, information presented in the content block 1 may be related to a degree of user attention. For example, information with a high degree of user attention in the display area is presented in the content block 1, and information with a low degree of user attention is not presented in the content block 1. The information with a high degree of user attention may be understood as an atlas, a table, a text, etc., that may characterize analysis results, and the information with a low degree of user attention may be understood as information unrelated to the analysis results on the analysis display interface, or information having a relatively low degree of correlation with the analysis results, or information that does not need to be presented in a medical report, for example, information of a module, which is located at the edge of the analysis display interface and used for assisting in the user operation.

The degree of attention may be set manually by the user, or may be set by an apparatus using the medical report generation method, which is not specifically limited in the present disclosure. In this way, information concerned by the user may be presented in the content block 1, and information less concerned by the user may be excluded automatically from the content block 1, thereby reducing the operation of manually removing the information with a low degree of attention by the user. Specifically, other information unrelated to the content block 1 may be contained on the analysis display interface, for example, the presentation of various operation modules (such as an analysis module and a database module), which facilitates the analysis by doctors. However, the doctors do not pay much attention to the information when interpreting analysis results, and the subject pays even less attention to the information, thus the redundant information may be excluded from the content block 1, so that the generated medical report is concise and clear, which facilitates people who obtain the report to interpret quickly the analysis results on the medical report without being interfered by the redundant information.

In some optional embodiments, information presented in the content block 1 may be related to a display type of information in the display area. For example, information presented in image type in the display area is presented in the content block 1, and/or information presented in table type in the display area is presented in the content block 1. Other information, if not presented in the form of image type or table type, then is not presented in the content block 1.

At step S104, a selection operation by the user with respect to at least one content block is received.

In some optional embodiments, upon the first extraction operation is received, each generated content block 1 is in a state to be selected, and the user may select freely a content block 1 that needs to be presented in the medical report. Then upon a selection operation performed by the user with respect to the content block 1 by means of an input device such as a mouse is received, the selected content block 1 may be presented in a selected state. Specifically, with reference to FIG. 3, a central node atlas, a functional connection atlas, and an eigenvalue table shown in FIG. 3 are each in a state to be selected. Further, if there is a "check mark" icon on a regional connection atlas in its middle part, indicating that the regional connection atlas is in the selected state (already selected state).

At step S105, a medical report is generated based on the at least one content block selected by the user.

In some optional embodiments, upon selecting a content block 1 to be selected, the user may perform a selection completion operation, thereby generating a medical report based on the selected content block 1. The above medical report may present clearly and completely the content of each selected content block 1, so that the user may obtain directly a medical report which is convenient for utilization, thereby improving the user's experience.

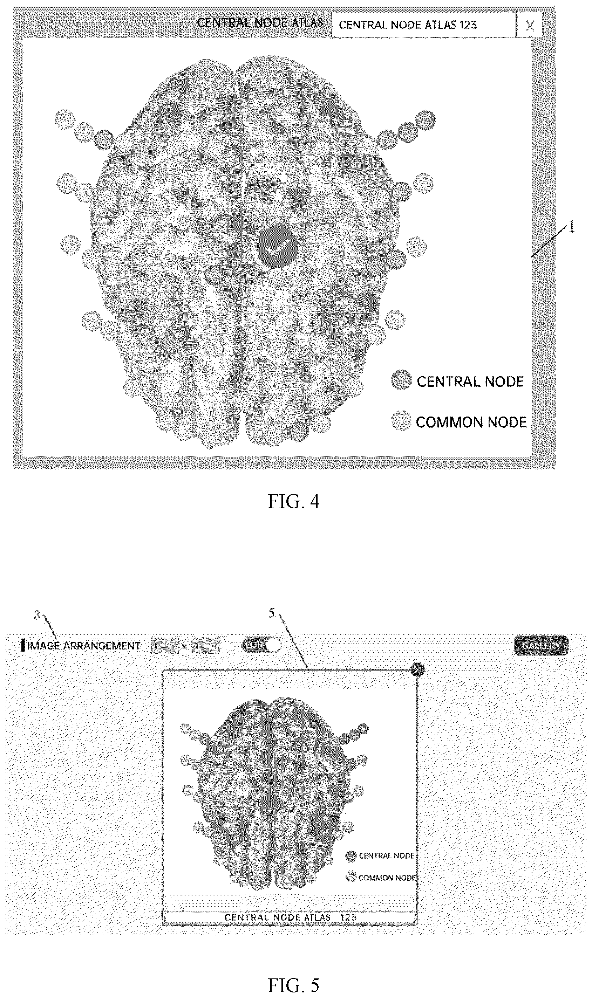

In some optional embodiments, the content of the content block 1 may at least contained an analysis result in a corresponding display area. As shown in FIG. 2 and FIG. 3, upon the first extraction operation with respect to the analysis display interface is received, the analysis display interface may be switched from an interface shown in FIG. 2 to an interface shown in FIG. 3, and the content blocks 1, which is presented automatically and prominently, just contains the above analysis results, but does not contain the titles corresponding to the respective areas. After at least one content block 1 selected by the user is acquired, it may proceed to a report editing interface shown in FIG. 5. What is presented on the report editing interface is not the content block 1 itself, instead, it is the content contained in the content block 1. Specifically, it can be understood as analysis results on the analysis display interface, such as the central node atlas shown in FIG. 5.

In some optional embodiments, the medical report may be divided into various forms of medical reports according to an arrangement manner, presentation content, etc.

In the present disclosure, after the first extraction operation by the user with respect to the analysis display interface is received, content block 1 can be generated automatically and a presented prominently. The content block 1 contains a corresponding analysis result, then a medical report is generated according to the selected content block 1, a doctor can obtain directly a required medical report based on the selected content block 1 through simple operations, without the need for manually repeating the operations. The medical report generation method has high universality, especially in the application of functional near-infrared spectroscopy field, reduces the difficulty in generating medical reports, simplifies operations while ensuring the doctors' degree of freedom of selection, and can reduce the information load during the doctor's operation, thereby facilitating the doctor's operation and use.

In some embodiments, the method may further include the following steps: a first selection operation for the content block 1 in the analysis display interface is received. In the analysis display interface, the related information of the content block 1 is presented in association with the content block 1 selected by the first selection operation.

In some optional embodiments, as shown in FIG. 4, after the user selects the content block 1 on the analysis display interface, a "check mark" icon is presented in the center of the content block 1, thereby characterizing that the content block 1 is in a selected state. Of course, other display manner may be used to identify that the content block 1 is in a selected state, such as shadow processing on the background where the selected content block 1 is located.

In some optional embodiments, the above related information may be one or more of a plurality of pieces of information such as title information, time domain information of near-infrared data corresponding to the content of the content block 1, etc., which is not specifically limited in the present disclosure. Upon receiving the first selection operation, the related information may be displayed automatically following the corresponding content block 1. For example, the related information is provided above or below the content block 1, etc., so that the user can acquire quickly the related information of the content block 1. For example, in FIG. 3, after the regional connection atlas is selected, its title information "regional connection atlas" is displayed automatically above the regional connection atlas.

In some optional embodiments, after the first selection operation with respect to the content block 1 is received, a close button is displayed on the selected content block 1, and the content block 1 may be deselected by clicking the close button. In some embodiments, the related information of the content block 1 is presented in an editable manner. In this way, the user may edit the related information of the content block 1 by himself/herself as required, with a certain degree of freedom. For example, the user may edit a title of the content block 1 by himself/herself.

In some optional embodiments, continuing with reference to FIG. 4, after the first selection operation is received, the title information associated with the content block 1 on the analysis display interface may be in an editable state, and the user may edit a title at the position of the title information. For example, title information "central node atlas" of the central node atlas in FIG. 4 may be modified to "central node atlas 123", and the modified title will be presented in the generated medical report following the selected central node atlas.

In some optional embodiments, the above medical report generation method may further include unedited related information of the content block 1 (that is, original related information of the content block 1 presented in a related manner when the content block 1 is selected by means of the first selection operation) and/or the already edited related information of the content block 1, which will be presented in the report editing interface following and in associated with the selected content block 1. In some embodiments, the related information of the above content block 1 may also be presented in the report editing interface in an editable manner, and the user may further edit the related information of the content block 1 by himself/herself as required in the report editing interface, with a certain degree of freedom. For example, in FIG. 4, the user modifies the title information "central node atlas" of the central node atlas to "central node atlas 123" in the analysis display interface. After entering the report editing interface, the user considers that the title "central node atlas 123" is inappropriate, at this moment, the "central node atlas 123" in the report editing interface is in an editable state, which provides the user with another opportunity to modify it, so that the user does not need to return to the analysis display interface to remodify the title information of the central node atlas, which is simple in operation and has high degree of freedom, improving sufficiently the user's usage experience. The above report editing interface may be understood as an interface for generating a medical report, into which the screen will enter after the content block 1 is selected on the analysis display interface.

In some embodiments, the step S105, i.e., generating a medical report based on the at least one content block 1 selected by the user, specifically includes: acquiring the at least one content block 1 selected by the user; and presenting content of the content block 1 in the report editing interface in a first preset arrangement manner. It can be understood that, the content of the selected content block 1 may be constructed as a filling block 5 to be filled into the report editing interface, and the filling blocks 5 are in one-to-one correspondence with the selected content blocks 1. For brevity, part of the content of the content block 1 will be expressed with the filling blocks 5 below.

It can be understood that, the above content block 1 may not be presented by being directly transferred to the report editing interface from the analysis display interface. In other words, what is presented on the report editing interface is not the content block 1, but the content contained in the content block 1. After the content block 1 is selected on the analysis display interface, the content contained in the selected content block 1 may be extracted. For example, data of an analysis result presented in the form of an image, text, and a table, etc., may be extracted. Taking the content block 1 as an HBO distribution graph (profile) as an example, the data extracted from and contained previously in the HBO distribution graph (profile) may be HBO data, title information of horizontal and vertical coordinates, etc. After the content contained in the content blocks 1 are extracted, filling blocks 5 in one-to-one correspondence with the content blocks 1 may be generated in the report editing interface. The filling blocks 5 are then filled into the report editing interface at the specified positions, so that the content of the content blocks 1, i.e. the filling blocks 5, may be presented on the report editing interface. Therefore, the matching between the content in the content block 1 and the report editing interface may be improved, the purpose of facilitating the user to obtain a clear and definite medical report is thus achieved.

In some optional embodiments, the first preset arrangement manner may be related to quantity, content, size, and the like of the selected content blocks 1. It can be understood that, only if the content blocks 1 selected by the user is acquired, the first preset arrangement manner may be generated. That is, the first preset arrangement manner is associated with the selected content blocks 1. In some other embodiments, the above first preset arrangement manner may also be unrelated to the selected content blocks 1, instead, the first preset arrangement manner may be selected/defined by the user, so as to generate a medical report that may satisfy the user requirements.

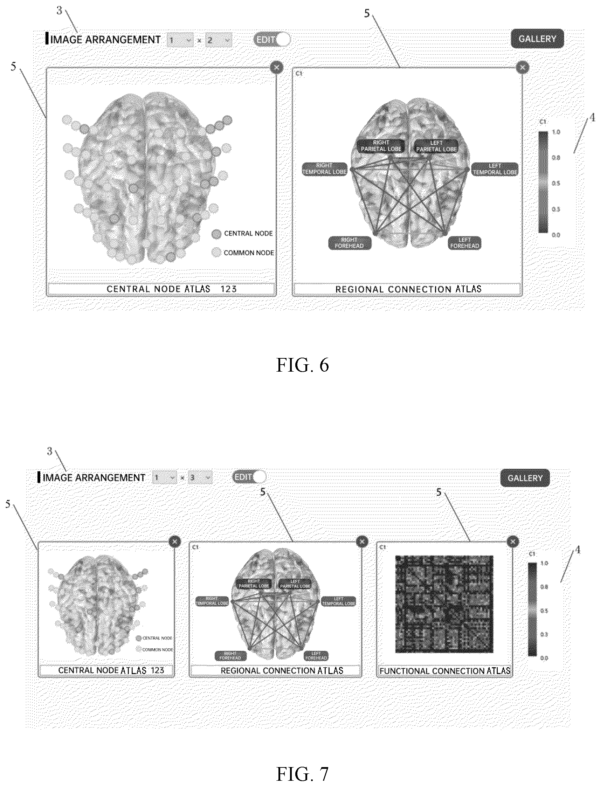

In some optional embodiments, as shown in FIG. 6 to FIG. 8, an image arrangement module 3 for setting the arrangement manner may be presented in the report editing interface, and the arrangement manner may be presented in the manner of "number of rows × number of columns", that is, filling blocks 5 in one-to-one correspondence with the content blocks 1 are arranged in rows and columns. In embodiments of the present application, a threshold may be defined for the number of rows and the number of columns in the above first preset arrangement manner. For example, the number of rows can be at most 2 rows, and the number of columns can be at most 3 columns. The above threshold may be determined according to the size of the interface, where the medical report is to be presented, so that each filling block 5 can achieve a better presentation effect on the report editing interface.

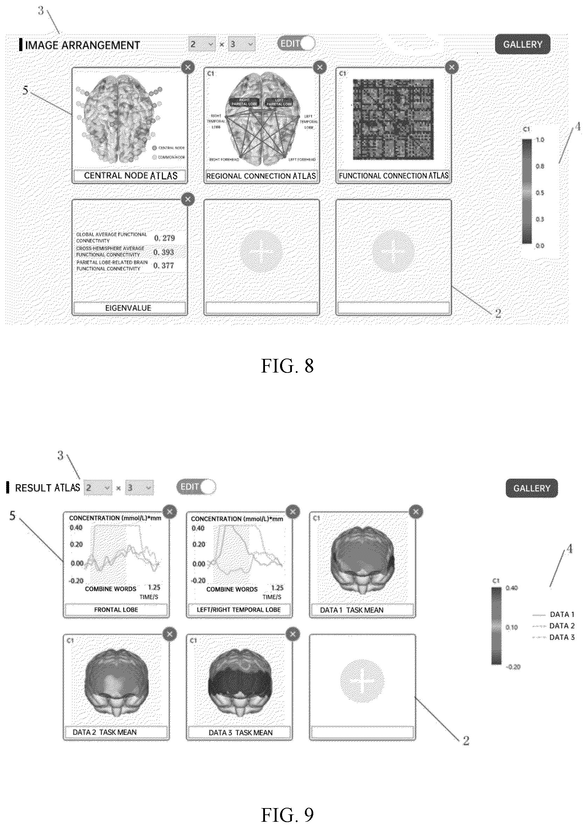

For example, as shown in FIG. 5 to FIG. 8, FIG. 5, FIG. 6, FIG. 7 and FIG. 8 show respectively the report editing interfaces, on which filling blocks 5 corresponding to the respective content blocks 1 are presented, when the user selects one, two, three, and four content blocks 1 on the analysis display interface. In this way, the filling blocks 5 can be presented in a corresponding and appropriate first preset arrangement manner according to the number of the selected content blocks 1. The first preset arrangement manner may vary with the change in the number or other attributes (such as size, display type, etc.,) of the selected content blocks 1, so as to obtain a clear and well-arranged medical report. Specifically, as shown in FIG. 5, when the number of the selected content block 1 is one, a first preset arrangement manner for presenting the filling block 5 on the report editing interface is "1×1". As shown in FIG. 6, when the number of the selected content blocks 1 is two, the first preset arrangement manner for presenting the filling blocks 5 on the report editing interface is "1×2". As shown in FIG. 7, when the number of the selected content blocks 1 is three, the first preset arrangement manner for presenting the filling blocks 5 on the report editing interface is "1×3". As shown in FIG. 8, when the number of the selected content blocks 1 is four, the first preset arrangement manner for presenting the filling blocks 5 on the report editing interface may be "2×3".

In some embodiments, the method further includes presenting the related information of the content blocks 1 and the content of the content blocks 1 in association with each other within the report editing interface. In some optional embodiments, the above related information, for example, may include title information, the time domain information of near-infrared data, etc. Within the report editing interface, the related information can be presented as following automatically the content display of the corresponding content block 1. For example, the related information is provided above or below the content block 1, etc., so that the user can acquire quickly the related information of the content block 1.

In some embodiments, the method may further include the following steps. A display type of the analysis result contained in content of each content block is identified. Wherein, the display type may include one or more of an image type and a table type. And the report editing interface may be divided into areas according to the identified display type of the analysis result, so that each divided area presents content of the content block 1 of the corresponding display type respectively.

In some optional embodiments, after the first preset arrangement manner is determined, an image display area and a table display area may be determined according to the display type of the analysis result on the report editing interface. After a selected content block 1 is acquired, based on the determined display type of the analysis result contained in the content of the content block 1, the content of the content block 1 corresponding to the image type is presented in the image display area, and the content of the content block 1 corresponding to the table type is presented in the table display area, so that each content may be displayed in a division manner according to the display type.

After the first extraction operation is received, the content of the content block 1 corresponding to the image type may be presented on the analysis display interface in the form of images. As a contrast, if the content of the content block 1 corresponding to the table type is also presented in the form of images, in some cases, the data contained in the content of the content block 1 of the table type may not be displayed clearly. For example, when the size of the table is too large, and data in the table is too much, etc., after the content of the content block 1 of the table type is filled into a box 2 to be filled in the report editing interface, the user will not be able to see the content in the table clearly. Taking this into consideration, the medical report generation method in embodiments of the present disclosure may further include the following steps.

The display type of the analysis result contained in content of each content block 1 may be identified, wherein the display type at least includes one or more of an image type and a table type.

When it is determined that the display type of the analysis result contained in the content of each content block 1 is the table type, table content of the content block 1 may be acquired.

A corresponding table object may be generated according to the table content, and the table object may be presented on the report editing interface.

In this way, by converting the table content into a corresponding table object, text, number and other content in the table object can be presented clearly on the report editing interface.

In some optional embodiments, after the table object is generated, the user's zoom-in operation or zoom-out operation with respect to the table object may be received to facilitate the user to further view the table object.

In some optional embodiments, after the table object is generated, a modification operation by the user with respect to the table object may be received to modify text, number and other content in the table object, thereby facilitating the user to modify the table object according to the actual situation.

In some embodiments, the content of the content block 1 is presented in a report editing interface in a first preset arrangement manner as follows. A plurality of boxes 2 to be filled are presented in the report editing interface according to the first preset arrangement manner. Appearance parameters of content of a corresponding content block 1 are adjusted based on display attributes of each of the boxes 2 to be filled, so that the content of the content block 1 is presented in a display manner adapted to the boxes 2 to be filled. The method further includes: based on the report editing interface, filling the content of the content block 1 with the adjusted appearance parameters into a corresponding box 2 to be filled, to generate a medical report containing the selected content block 1. In this way, the content contained in the above content block 1 may be filled fully and properly in the box 2 to be filled, so that the content corresponding to the content block 1 may be better presented in the box 2 to be filled.

In some optional embodiments, display attributes of the above box 2 to be filled may be understood as size, color, shape, etc., of the box 2 to be filled, and appearance parameters of the content of the content block 1 may be understood as size, color, and resolution of the content of the content block 1. For example, after the content block 1 is selected, text, images and other data information contained in the content of the content block 1 may be extracted, and the text contained in the content of the selected content block 1 may be zoomed in, zoomed out or bolded on the report editing interface, so that the text contained in the content block 1 can be presented clearly in the box 2 to be filled of the report editing interface. For another example, the image contained in the content of the selected content block 1 may be zoomed in or zoomed out on the report editing interface, so that the image contained in the content block 1 can be presented clearly in the box 2 to be filled of the report editing interface. In this way, by means of improving the adaptability between the content of the content block 1 and the box 2 to be filled, a clearer medical report can be obtained.

The size, color and other attributes of the respective boxes 2 to be filled on the report editing interface may be the same or different from each other. When the content of each content block 1 is presented on the report editing interface, the above appearance parameters of the content of the content block 1 may be adjusted according to the attributes of the box 2 to be filled therein. For example, the size of the content of the content block 1 is adjusted according to the size of the box 2 to be filled, so that the content contained in the content block 1 may fill and fit the box 2 to be filled, and presented in the box 2 to be filled with an optimal display effect. For example, the resolution of the content of the content block 1 is adjusted according to the size of the box 2 to be filled, so that the content of the content block 1 may be presented more clearly. For another example, the color of the content of the content block 1 is adjusted according to the color of the box 2 to be filled, so the content of each content block 1 may be presented with a color of a corresponding box 2 to be filled as the background color, such that the user may determine a corresponding relationship between the content of each content block 1 and the box 2 to be filled.

As shown in FIG. 8, the above box 2 to be filled is used to fill the content of the selected content block 1 therein, so as to present the content of each content block 1 on the report editing interface. In some optional embodiments, when the number of selected content blocks 1 does not reach the number of boxes 2 to be filled corresponding to the first preset arrangement manner, a box 2 to be filled in an unfilled state may be presented on the report editing interface. For example, as shown in FIG. 8, four content blocks 1 are selected. In this case, the first preset arrangement manner may be "2×3", that is, there are totally six boxes 2 to be filled. Therefore, there are two boxes 2 to be filled in an unfilled state presented on the report editing interface as shown in FIG. 8.

In some optional embodiments, after adjusting appearance parameters of the content of each content block 1 to fill in the box 2 to be filled, the user may generate a medical report based on the selected content block 1 by clicking a Generate Report button. The above medical report may present clearly and completely the content of each selected content block 1, enabling the user to obtain directly the easy-to-use medical report, thereby improving the user's experience.

In some optional embodiments, the above medical report may adopt various division forms according to an arrangement manner, presentation content, etc.

In the present disclosure, the content of the selected content block 1 is presented in a first preset arrangement manner on the report editing interface, and the content of the content block 1 with adjusted appearance parameters is filled automatically in a corresponding box 2 to be filled to generate the medical report, so that doctors are able to present clearly and completely the content of each content block 1 in the medical report upon selecting the content block 1. That is to say, a medical report tailored to doctors' needs can be generated, which is especially beneficial to the situation where a plurality of analysis results can be selected out and displayed, without the need for doctors to repeat manually the operations, which is not only universal but also convenient.

In some embodiments, the method further includes matching out a corresponding arrangement manner within the report editing interface according to the number of the selected content blocks 1.

In some optional embodiments, if the number of the selected content blocks 1 is determined, then an arrangement manner matching the number may be obtained directly. It should be noted that, the arrangement manner may be matched automatically without needing manual selection by the user. For example, as shown in FIG. 6 to FIG. 10 and FIG. 12, FIG. 6, FIG. 7, FIG. 10 and FIG. 12 show respectively the report editing interfaces, on which filling blocks 5 corresponding to the selected content blocks 1 are presented, when the user selects two, three, four and five content blocks 1. In this way, each filling block 5 may be presented in a corresponding and appropriate arrangement manner according to the number of the content blocks 1. The arrangement manner may vary with the change in the number or other attributes (such as size and display type) of the selected content blocks 1, so as to obtain a clear and well-arranged medical report.

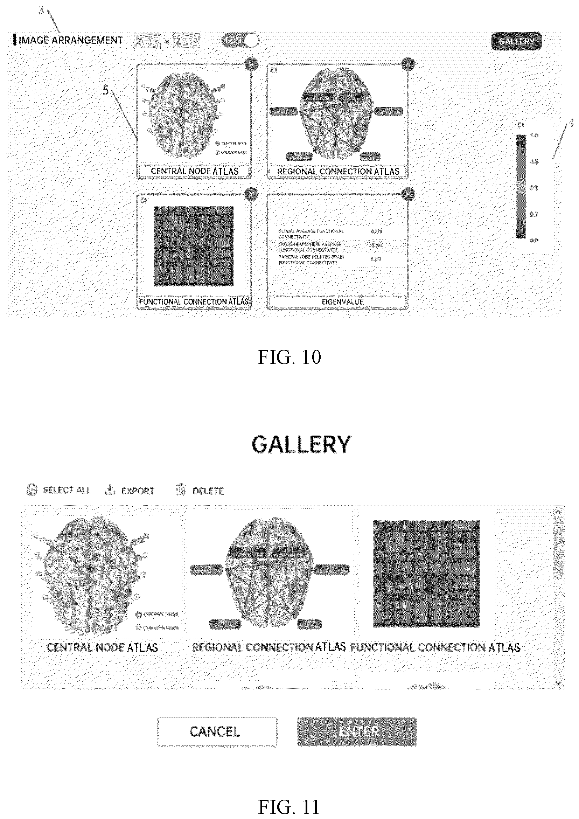

Specifically, as shown in FIG. 6, when the number of the content blocks 1 is two, the arrangement manner for presenting the filling blocks 5 on the report editing interface is "1×2". As shown in FIG. 7, when the number of the content blocks 1 is three, the arrangement manner for presenting the filling blocks 5 on the report editing interface is "1×3". Specifically, for the number of the content blocks, the arrangement manner may be set by taking a minimum number of rows and a minimum number of columns which may accommodate all the content blocks as criteria. As shown in FIG. 10, when the number of the content blocks 1 is four, the arrangement manner for presenting the filling blocks 5 on the report editing interface is "2×2". As shown in FIG. 12, when the number of the content blocks 1 is five, the arrangement manner for presenting the filling blocks 5 on the report editing interface is "2×3".

In some embodiments, the method further includes: upon re-entering an analysis display interface and re-selecting content blocks 1, a corresponding arrangement manner, in which the re-selected content blocks 1 are presented on the report editing interface, is matched based on the re-selected content blocks 1. In this way, the arrangement manner varies with the change in the selected content blocks 1, so that the arrangement manner can be adjusted automatically according to the selected content blocks 1. In some embodiments, upon the corresponding arrangement manner, in which the re-selected content blocks 1 are presented on the report editing interface, are matched for the re-selected content blocks 1, display attributes of each box 2 to be filled may also vary with the number of the re-selected content blocks 1, that is, when the number of the selected content blocks 1 varies, display attributes of the box 2 to be filled may also be adjusted, and appearance parameters of the content of the corresponding content blocks 1 may be adjusted based on the adjusted display attributes of the boxes 2 to be filled, so that a clear medical report can be generated. For example, when the content blocks 1 selected for the first time are two profiles, the number of boxes 2 to be filled shown on the report editing interface is two, and fonts of horizontal and vertical coordinates in the two profiles are presented respectively in the first preset size in the corresponding boxes 2 to be filled respectively. Upon re-entering the analysis display interface and re-selecting two content blocks 1, the number of the boxes 2 to be filled shown on the report editing interface is four, which are used to present the two previously selected profiles and the content of the two re-selected content blocks 1 respectively. In this case, the fonts of the horizontal and vertical coordinates in the two profiles may be presented in a second preset size larger than the first preset size in the boxes 2 to be filled, so that the key information of the content of the content blocks 1 can still be presented clearly to the user, facilitating the user to interpret the medical report.

In some optional embodiments, after the content blocks 1 are re-selected, it may be determined whether the number of the selected content blocks 1 has changed. If so, a corresponding arrangement manner may be re-matched for the already changed number of content blocks 1; otherwise, the re-selected content blocks 1 may be presented in the previous arrangement manner adopted before the content blocks 1 are re-selected on the report editing interface.

In some embodiments, the method further includes acquiring the order of selecting content blocks 1, and presenting successively the content of respective content blocks 1 on the report editing interface in reverse order. In this way, the content of content blocks 1 recently selected by the user may be placed in front of the content of content blocks 1 previously selected, thus facilitating the user to view directly the content of the recently selected content blocks 1.

For example, the user may select a functional connection atlas, a regional connection atlas and a central node connection atlas in order, so that upon entering the report editing interface, as shown in FIG. 7, the functional connection atlas, the regional connection atlas and the central node connection atlas on the report editing interface may present the content of the respective content blocks 1 in reverse order, that is, the central node connection atlas is placed in front of the regional connection atlas, and the regional connection atlas is placed in front of the functional connection atlas.

In some other embodiments, the method further includes acquiring the order of selecting the content blocks 1, and presenting the content of the respective content blocks 1 in order.

In some embodiments, the method further includes the following steps.

An image arrangement module 3 for adjusting an arrangement manner may be presented on the report editing interface. In response to a modification operation on the arrangement manner of the image arrangement module 3, the boxes 2 to be filled may be presented on the report editing interface in a modified arrangement manner.

In some optional embodiments, continuing with reference to FIGS. 6 to 8 and FIG. 10, after a corresponding arrangement manner is matched for the content blocks 1, an image arrangement module 3 corresponding to the arrangement manner may be presented on the report editing interface. A current arrangement manner is presented in the form of "number of rows × number of columns" on the image arrangement module 3. The current arrangement manner may be modified based on the modification operation on the image arrangement module 3, so that the report editing interface may present content of the content blocks 1 in a modified arrangement manner.

For example, as shown in FIG. 8 and FIG. 10, a number of filling blocks 5 on the report editing interface shown in FIG. 10 is four, and the arrangement manner is "2×2". After a modification operation of modifying "2×2" to "2×3" on the image arrangement module 3 is received, the arrangement manner on the report editing interface shown in FIG. 10 is switched to the arrangement manner on the report editing interface shown in FIG. 8, that is, the arrangement manner is modified to "2×3". In this way, a corresponding arrangement manner can be matched automatically according to the number of selected content blocks 1, and the matched arrangement manner can also be modified according to user requirements, so as to facilitate operation and use by the user.

In some optional embodiments, the above image arrangement module 3 may have a locked state and an unlocked state. In the locked state, the arrangement manner on the image arrangement module 3 cannot be modified, while in the unlocked state, it may be allowed to modify the arrangement manner on the image arrangement module 3, so that mistaken operation on the image arrangement module 3 can be avoided.

In some embodiments, the method further includes determining the content of the content blocks 1 filled in the boxes 2 to be filled based on an order of selecting the content blocks 1, when the number of the selected content blocks 1 exceeds a preset threshold. Specifically, when the number of selected content blocks 1 exceeds the preset threshold, the content of the content blocks 1 filled in the boxes 2 to be filled may be determined based on the order of selecting the content blocks 1. For example, the content of the content blocks 1 filled in the boxes 2 to be filled is determined in reverse order. The content of the recently selected content blocks 1 are filled in the boxes 2 to be filled, and the content of the content blocks 1, which cannot be filled in the boxes 2 because of the lack of the number of the boxes 2, are stored in a picture library (gallery). For example, the maximum arrangement manner of the image arrangement module 3 is "2×3", that is, there may be a maximum of 6 boxes 2 to be filled to present content of up to six content blocks 1. When the number of content blocks 1 selected by the user exceeds six, for example, when seven content blocks 1 are selected, a selection order of the seven content blocks 1 may be obtained, content of a content block 1 selected firstly may be stored in a picture library, and content of the six content blocks 1 selected later may be presented in six boxes 2 to be filled respectively. In some other embodiments, optionally, the content of the last selected content block 1 may be stored in the picture library.

In this way, the user may screen initially content of candidate content blocks 1, and the number may exceed a preset threshold (for example, seven content blocks 1). The content of the candidate content blocks 1 may be stored in the picture library, so that the user may check, screen or adjust the selected content blocks 1 as required, and finally determine target content blocks used for generating the medical report.

In some embodiments, the picture library may also store content of all the content blocks 1 selected by the user, so that the user may screen or adjust the selected content blocks 1 as required, without needing to re-enter the analysis display interface to re-acquire the content blocks 1.

In some optional embodiments, the preset threshold may be set automatically by a system or an apparatus using the medical report generation method, or may be customized by the user, which is not specifically limited in the present disclosure.

In some optional embodiments, when the number of the selected content blocks 1 exceeds the preset threshold, the content of the content blocks 1 presented on the report editing interface, the number of which satisfies the preset threshold, are related to the order of selecting the content blocks 1. For example, when the preset threshold is six, the content of six content blocks 1 recently selected are arranged on the report editing interface, and the content of all content blocks 1, including the content of the above six content blocks 1, are stored in the picture library.

In some optional embodiments, the medical report generation method may further include presenting a picture library icon associated with the picture library on the report editing interface. And in response to a selection operation for selecting the picture library icon, the stored content of the content blocks 1 are presented on a picture library interface corresponding to the picture library.

In some embodiments, the method may further include: in response to a sorting operation for adjusting the arrangement order of the content of the content blocks 1 on the picture library interface, presenting the content of the content blocks 1 in an adjusted arrangement order on the report editing interface.

In some optional embodiments, the interface shown in FIG. 11 is a picture library interface. After a click operation on the picture library icon on the report editing interface is received, the picture library interface will be presented. The content of all the content blocks 1 are stored in the picture library interface, and the arrangement order of the content of the content blocks 1 may be related to a selection order of the user. Specifically, the content of the content blocks 1 may be arranged in reverse order of the selection order of the user on the picture library interface.

Specifically, the sorting operation may be understood as an adjustment operation on the arrangement order of the content of the content blocks 1 on the picture library interface, and may be specifically implemented by means of an operation of dragging the content of the content blocks 1 to the vicinity of the content of other content blocks 1, or may be implemented by editing position attributes of the content of the content blocks 1. The specific operation manner of the arrangement operation is not specifically limited in the present disclosure, as long as the order of the content of content blocks 1 may be adjusted.

For example, in case that the above preset threshold is six, the content of all the content blocks 1 will be stored on the picture library interface when the number of the selected content blocks 1 exceeds six. In this case, the picture library interface may be presented by clicking the picture library icon. The content of the first six content blocks 1 presented on the picture library interface are the content of six content blocks 1 that will be displayed on the report editing interface, the content of other content blocks 1 will not be displayed on the report editing interface. The content of the content blocks 1 behind the six content blocks 1 are the content of the content blocks 1, which are selected by the user but not presented on the report editing interface. The content of the adjusted first six content blocks 1 may be presented on the report editing interface by adjusting the arrangement order of the content of the content blocks 1. In this way, the user may adjust the content of the content blocks 1 that he/she wants to display on the report editing interface as required, without needing to re-enter the analysis display interface to re-select the content blocks 1, which is convenient to operate and use.

In some embodiments, the method further may include the following steps.

The picture library interface is presented in response to a selection operation on the box 2 to be filled on the report editing interface.

The content of the content blocks 1 selected on the picture library interface are acquired, and the content of the content blocks 1 may be presented in the boxes 2 to be filled. Specifically, appearance parameters of the content of the content blocks 1 selected on the picture library interface may be adjusted based on the display attributes of the boxes 2 to be filled, so that a clear medical report may be generated. For example, when the content of the content block 1 selected on the picture library interface is a profile, font size of the horizontal and vertical coordinates, profile color and the like in the profile may be adjusted according to the display attributes of the box 2 to be filled.

In some optional embodiments, an addition button for adding the content of the content block 1 may be provided in the above box 2 in an unfilled state, for example, "+" button shown in FIG. 9. By clicking the addition button, the picture library interface may be presented to add the content of the content blocks 1 stored in the picture library to the box 2 to be filled. It can be understood that the picture library may be presented based on any operation. For example, when it is detected that the mouse is hovering in the box 2 to be filled, the picture library may be presented automatically, making the operation more convenient.

In some optional embodiments, after the picture library interface is presented, the content of the content block 1, which has been presented on the report editing interface, may be identified on the picture library interface, so the content is displayed differently from the content of the content block 1, which is not presented on the report editing interface, such that the user may know clearly the content of the content block 1 that has been displayed on the report editing interface. Of course, the user may also re-select the content of the content block 1 that has been presented on the report editing interface, so that the content block(s) 1 containing the same content may be presented in different boxes 2 to be filled on the report editing interface.

In some embodiments, the method may further include the following steps.

Reference information 4 presented in the display area and located outside the content block 1 corresponding to the display area may be determined, wherein the reference information 4 is used to assist the user in interpreting the analysis result.

It may be judged whether the reference information 4 corresponds to a plurality of analysis results presented on the report editing interface.

If so, the reference information 4 is not presented repeatedly on the report editing interface, and the reference information 4 is presented in association with content blocks 1 having the analysis results corresponding thereto.

If not, each piece of reference information 4 is presented one by one on the report editing interface, and the reference information 4 is presented in association with the content of content blocks 1 having the analysis results corresponding thereto.

In this way, it is possible to avoid presenting repeatedly the reference information 4 containing the same content on the medical report, thereby reducing the space proportion of the reference information 4 on the medical report, maintaining the medical report as being concise and clear, and facilitating the user to interpret the medical report. In some embodiments, the reference information 4 is not located in the content block 1, which can reduce the area proportion of the boxes 2 to be filled on the report editing interface, so that the content contained in the content block 1 can fill the box 2 to be filled to a sufficient extent, so as to present clearly the content of the content block 1 in the box 2 to be filled.

In some optional embodiments, the above reference information 4 may include indication information corresponding to a curve on a graph, color block (hotmap scale) information corresponding to an atlas, etc., as long as the reference information 4 can be used to assist the user in interpreting an analysis result in the content block 1, which is not specifically limited in the present disclosure. Specifically, with reference to FIG. 9, in case that the content of the content block 1 includes the atlas, the reference information 4 at least includes color block information corresponding to the atlas.

For example, as shown in FIG. 9, "DATA 1", "DATA 2" or "DATA 3" shown on the right side in FIG. 9 is indication information corresponding to the graphs, and color block information shown on the right side in FIG. 9 is reference information 4 corresponding to a task mean graph of the data 1, a task mean graph of the data 2 and a task mean graph of the data 3.

In some optional embodiments, the above reference information 4 may have a corresponding relationship with analysis results. For example, when the analysis results include a atlas, the analysis results may have a corresponding relationship with reference information 4 containing color block information, and when the analysis results include a graph, the analysis results may have a corresponding relationship with reference information 4 containing indication information. The indication information may be used to identify a curve in the graph.

In some optional embodiments, some of the analysis results correspond to reference information 4, and some of the analysis results do not correspond to the reference information 4. Before judging whether the reference information 4 corresponds to the analysis results contained in the content of a plurality of content blocks 1 presented on the report editing interface, the plurality of content blocks 1 corresponding to the reference information 4 may be determined first. By judging repeated reference information 4 and non-repeated reference information 4 among the plurality of content blocks 1, it is determined that the repeated reference information 4 is not presented repeatedly on the report editing interface, and the non-repeated reference information 4 is presented one by one.

In some optional embodiments, upon the first extraction operation by the user with respect to the analysis display interface is received, the generated content block 1 presented on the analysis display interface does not contain the above reference information 4. Then, after the analysis display interface is switched to the report editing interface, the reference information 4 corresponding to the content of the selected content block 1 will be presented as following automatically the content of the content block 1 on the report editing interface, facilitating users to read analysis results in combination with the content of the content block 1 and the reference information 4.

In some embodiments, the method may further include the following steps.

A response may be made to an arrangement operation on the report editing interface, which is used for adjusting an arrangement position of content of the content blocks 1.

An arrangement position of the reference information 4 may be adjusted on the report editing interface based on the arrangement operation.

In some optional embodiments, the above arrangement operation may be understood as an adjustment operation with respect to an arrangement order of the content of the content blocks 1 on the report editing interface, and may be specifically implemented by means of an operation of dragging the content of the content blocks 1 to the vicinity of the content of other content blocks 1, or may be implemented by editing position attributes of the content of the content blocks 1. The specific operation manner of the arrangement operation is not specifically limited in the present disclosure, as long as the order of the content of the content blocks 1 can be adjusted.

For example, as shown in FIG. 9, in case that atlases and graphs are presented on a first row of the report editing interface, and atlases are presented on a second row, indication information corresponding to a curve on the graphs and color block information corresponding to the atlases may be both displayed on the right side in the center height. In FIG. 12, when only graphs are presented on a first row of the report editing interface and only atlases are represented on a second row, indication information corresponding to a curve on the graphs may be displayed to the right and close to the first row, and color block information corresponding to the atlases may be displayed to the right and close to the second row. In this way, the reference information 4 varies with a position change of the content of the content block 1 corresponding thereto, so as to facilitate the user to read a generated medical report.

In some embodiments, presentation of the reference information 4 in association with the content of content blocks 1 having the analysis results corresponding thereto specifically includes setting the same identification feature for the reference information 4 and the content of the content blocks 1 having the analysis results corresponding thereto.

In some optional embodiments, the above identification feature may be text, icon, etc., and the identification feature may be provided respectively at a preset position of the content of the content blocks 1 (i.e., a preset position of a filling block 5), and a preset position of the reference information 4. For example, as shown in FIG. 7, the reference information 4 is color block information. An identification feature "C1" is presented on the color block information, and the same identification feature "C1" is presented at the upper left corner of the filling block 5 corresponding to the color block information. In this way, the user may interpret a regional connection atlas and a functional connection atlas by referring to the color block information, which can reduce the space proportion of the reference information 4 on the medical report, and can facilitate the user to read information.

In some optional embodiments, the above medical report generation method may further include receiving a display operation with respect to the reference information 4, and in response to the display operation, presenting the zoomed-in or zoomed-out reference information 4 on the report editing interface.

In some embodiments, the method may further include the following steps.

A second extraction operation by the user with respect to the analysis display interface is received.

In response to the second extraction operation, at least one of a plurality of content blocks 1 is presented on a template interface, wherein each content block 1 presented in the template interface is in a selected state.

In this way, the content blocks 1 may be extracted directly without requiring the user to select some of the content blocks 1 in order to be presented on the report editing interface from the plurality of content blocks 1, so that the medical report may be generated conveniently and quickly.

Specifically, the above second extraction operation is an operation different from the first extraction operation. Another extraction module different from an extraction module corresponding to the first extraction operation may be presented on the analysis display interface, and this another extraction module corresponds to the second extraction operation. Upon the user selects this another extraction module by means of an input device such as a mouse, the second extraction operation may be generated. In some other embodiments, the above second extraction operation may also be generated by operations such as triggering of a shortcut key, etc. The method for generating the second extraction operation is not limited in the present disclosure, as long as it facilitates the user to generate the second extraction operation.

In some optional embodiments, the second extraction operation may be understood as extracting the content blocks 1 directly and automatically without requiring the user to select, successively in order, some of the content blocks 1 to be presented on the report editing interface from the plurality of content blocks 1.

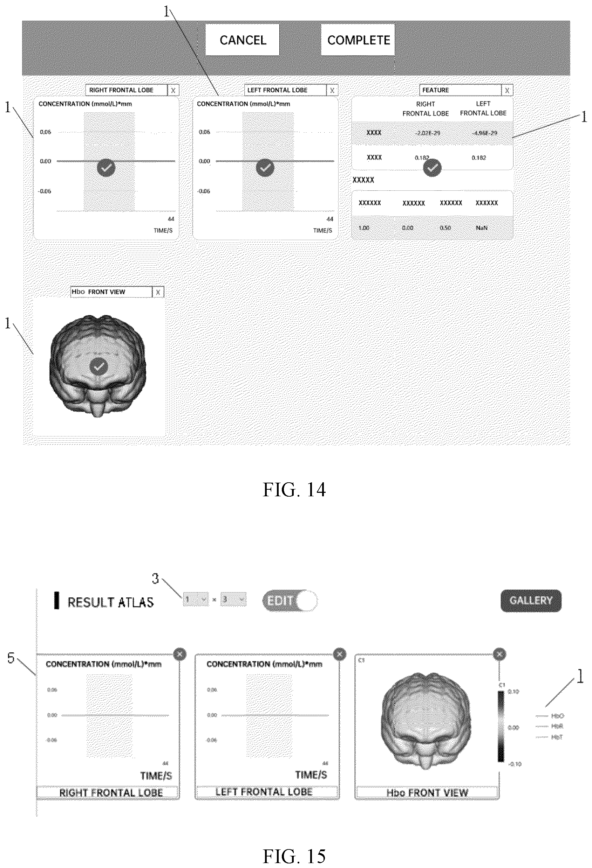

For example, as shown in FIG. 13 and FIG. 14, "second extraction block" shown in FIG. 13 may be understood as another extraction module corresponding to the second extraction operation, and if the user selects the "second extraction block", then the above second extraction operation may be generated. The interface shown in FIG. 14 is a template interface, and in response to the second extraction operation, the template interface may be presented, and each content blocks 1 in the template interface is in a selected state in an automatic manner, so that the user does not need to select manually the individual content block 1, which facilitates the user to implement a convenient and quick operation.

In some optional embodiments, as shown in FIG. 14, a "complete" button may be presented on the template interface, only if the user selects the "complete" button, it may be presented directly on the report editing interface. As shown in FIG. 15 (in which the table content in FIG. 14 is not shown), each content block 1 presented in the template interface may be presented in the report editing interface, so that the selection of the content blocks 1 to be presented on the report editing interface may be achieved by a single operation of one key-press, and the user does not need to select manually the respective content blocks 1 one by one in order.

In some embodiments, in response to the second extraction operation, at least one of a plurality of content blocks 1 is presented on the template interface, specifically including the following steps.

A first configuration manner for configuring the current blocks 1 on the template interface is determined.

At least part of the plurality of content blocks 1 on the analysis display interface are determined as target content blocks based on the first configuration manner, and it is determined that the target content blocks are presented on the template interface in a second preset arrangement manner.

In this way, the content blocks 1 that need to be presented on the report editing interface can be configured directly for the user through the first configuration manner, and the arrangement manner of the content blocks 1 can be determined directly, so the user does not need to select manually the content blocks 1 one by one in order, nor does he/she need to select the arrangement manner, thus the user can obtain the medical report based on fewer operations, achieving convenient operation.

Specifically, the first configuration manner may be understood as being used for determining at least part of the plurality of content blocks 1 on the analysis display interface as target content blocks, and determining a second preset arrangement manner in which the target content blocks are presented on the report editing interface. The target content blocks corresponding to the first configuration manner may be related to diseases, that is, for different diseases, the target content blocks to be presented on the report editing interface determined in the first configuration manner may be different.

Specifically, the method for determining the first configuration manner may specifically include one of determining a default configuration manner, determining a configuration manner selected by the user last time and presenting a configuration manner selection list for the user, and receiving the user's selection operation for selecting the first configuration manner from the configuration manner selection list.

In some embodiments, the method further includes receiving an adjustment operation by the user with respect to the first configuration manner on the template interface; in response to the adjustment operation, generating a second configuration manner corresponding to the adjustment operation; and presenting a selection box capable of switching the first configuration manner to the second configuration manner on the template interface.

In this way, the target content blocks indicated by the above first configuration manner and the arrangement manner of the target content blocks may be adjusted by the user, and may vary as desired. After the user adjusts the first configuration manner to generate the second configuration manner, the user may further switch the first configuration manner to the second configuration manner, so that when the second extraction operation by the user with respect to the analysis display interface is received next time, it can be determined that the content blocks 1 are configured on the template interface in the second configuration manner.

Specifically, the above adjustment operation includes at least one of: an operation of adjusting at least one content block 1 in a selected state presented in the template interface to a deselected state; and an operation of modifying an arrangement manner of the corresponding content blocks 1.

For example, after generating the second configuration manner and before entering the report editing interface, a selection box indicating "whether to set a current configuration manner as a default configuration manner" may be presented on the template interface, and if the user selects yes, the first configuration manner is switched to the second configuration manner; if the user selects no, the setting of configuring content block 1 on the template interface in the first configuration manner is maintained.

In some embodiments, after a selection operation by the user with respect to at least one content block 1 is received, the method further includes the following steps.

A third configuration manner corresponding to the selection operation is generated based on the selection operation by the user with respect to the at least one content block 1, wherein the third configuration manner is used for determining at least part of the plurality of content blocks 1 on the analysis display interface as target content blocks, and it is determined that the target content blocks are presented in a third preset arrangement manner on the report editing interface.

Specifically, prompt information for saving the third configuration manner may also be presented.

In this way, after the user selects the required content blocks 1, a third configuration manner corresponding to the user's selection operation may be matched for the user. The third configuration manner may be saved, so that when the user selects the content block 1 that needs to be presented on the report editing interface, the content block 1 may be configured and presented directly on the template interface in the third configuration manner, or configured and presented directly on the report editing interface, further achieving convenient operations.

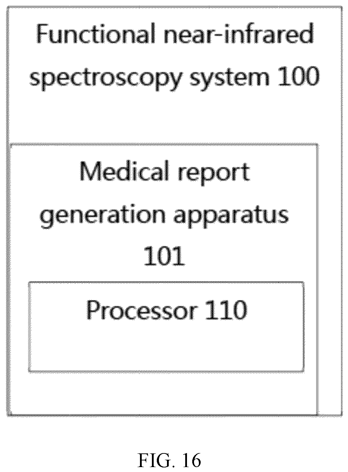

An embodiment of the present disclosure further provides a medical report generation apparatus 101. As illustrated in FIG. 16, the medical report generation apparatus 101 at least includes a processor 110. The processor 110 is configured to perform the medical report generation method of any of the above embodiments. The medical report generation apparatus 101 using the above medical report generation method, after receiving the first extraction operation by the user with respect to the analysis display interface, can generate automatically a prominently presented content block 1. The content block 1 contains a corresponding analysis result, and a medical report is generated according to the selected content block 1, thus a doctor may acquire directly a required medical report based on the selected content block 1 through simple operations, without the need for manual repeating of the operations. The medical report generation apparatus 101 has high universality in the application of near-infrared data, reduces the difficulty in generating medical reports, simplifies operations while ensuring the doctors' degree of freedom of selection, and reduces the information load during the doctor's operation, thereby facilitating the doctor's operation and use.

An embodiment of the present disclosure further provides a storage medium storing a computer program, wherein the computer program, when executed by a processor, implements the medical report generation method of each of the embodiments of the present disclosure.

An embodiment of the present disclosure further provides a functional near-infrared spectroscopy system 100. As shown in FIG. 16, the functional near-infrared spectroscopy system 100 at least includes the above medical report generation apparatus 101. The functional near-infrared spectroscopy system 100 using the above medical report generation apparatus 101, after receiving the first extraction operation by the user with respect to the analysis display interface, can generate automatically a prominently presented content block 1. The content block 1 contains a corresponding analysis result, and a medical report is generated according to the selected content block 1. In this way, a doctor can acquire directly a required medical report based on the selected content block 1 through simple operations without the need for manual repeating of the operations. The functional near-infrared spectroscopy system 100 has high universality in the application of near-infrared field, reduces difficulty in generating medical reports, simplifies operations while ensuring the doctors' degree of freedom of selection, and reduces the information load during the doctor's operation, thereby facilitating the doctor's operation and use.

Note that each unit in each embodiment according to this application can be implemented as computer executable instructions stored in the memory (or storage), and the corresponding steps can be implemented when the instructions are executed by the processor. It can also be implemented as hardware with corresponding logic computing capability. It can also be implemented as a combination of software and hardware (firmware). In some embodiments, the processor can be implemented as any of CPU, FPGA, ASIC, DSP chip, SOC (system on chip), MPU (such as but not limited to Cortex), etc. A processor may be communicatively coupled to a memory (or storage) and configured to execute computer executable instructions stored therein. The memory (or storage) may include read-only memory (ROM), flash memory, random access memory (RAM), dynamic random-access memory (DRAM) such as synchronous DRAM (SDRAM) or Rambus DRAM, static memory (such as flash memory, static random-access memory), etc., on which computer executable instructions are stored in any format. The computer executable instructions can be accessed by the processor, read from ROM or any other suitable storage location, and loaded into RAM for the processor to execute, so as to realize the medical report generation method according to various embodiments of the present application.

It should be noted that in each component of the system of this application, the components are logically divided according to the function to be realized. However, the application is not limited to this, and each component can be redivided or combined as needed. For example, some components can be combined into a single component, or some components can be further decomposed into more subcomponents.

Each component embodiment of this application can be implemented in hardware, or in software modules running on one or more processors, or in their combination. Those skilled in the art should understand that a microprocessor or a digital signal processor (DSP) can be used in practice to realize some or all functions of some or all components of the system according to the embodiments of the present application. The present application may also be implemented as an apparatus or device program (e. g., a computer program and a computer program product) for executing part or all of the methods described herein. Such a program implementing the present application may be stored on a computer-readable medium, or may be in the form of one or more signals. Such signals can be downloaded from Internet websites, or provided on carrier signals, or provided in any other form. In addition, the present application may be implemented by means of hardware including several different elements and by means of a properly programmed computer. In the unit claims listing several devices, several of these devices may be embodied by the same hardware item. The use of words first, second, and third does not indicate any order. These words can be interpreted as names.

Furthermore, although exemplary embodiments have been described herein, their scope includes any and all embodiments having equivalent elements, modifications, omissions, combinations (e. g., schemes crossed by various embodiments), adaptations or changes based on the present application. The elements in the claims will be interpreted broadly based on the language used in the claims, not limited to the examples described in the description or during the implementation of this application, and the examples will be interpreted as non-exclusive. Therefore, the specification and examples are intended to be considered as examples only, and the true scope and spirit are indicated by the following claims and the full scope of their equivalents.

The above description is intended to be illustrative rather than restrictive. For example, the above examples (or one or more of them) may be used in combination with each other. For example, those skilled in the art may use other embodiments when reading the above description. In addition, in the above specific embodiment, various features can be grouped together to simplify this application. This should not be interpreted as the intention that a public feature that does not require protection is necessary for any claim. On the contrary, the subject matter of the present application may be less than all the features of a particular disclosed embodiment. Thus, the following claims are hereby incorporated into the specific embodiments as examples or embodiments, wherein each claim is independently regarded as a separate embodiment, and it is considered that these embodiments can be combined with each other in various combinations or arrangements. The scope of the present application shall be determined by reference to the full scope of the appended claims and the equivalent forms empowered by these claims.

The above embodiments are only exemplary embodiments of this application and are not intended to limit this application. The protection scope of the application is defined by the claims. Those skilled in the art may make various modifications or equivalent substitutions to this application within the substance and protection scope of this application, and such modifications or equivalent substitutions shall also be deemed to fall within the protection scope of the application.

## Claims

1. A medical report generation method, **characterized in that**, comprising:
presenting an analysis display interface based on medical data, so that at least one display area, which is used for displaying a corresponding analysis result, is distributed on the analysis display interface;
receiving a first extraction operation by a user with respect to the analysis display interface;
in response to the first extraction operation, generating content blocks corresponding to the respective analysis results and presenting prominently the content blocks on the analysis display interface, wherein the content blocks are provided in one-to-one correspondence with the display areas on the analysis display interface;
receiving a selection operation by the user with respect to at least one content block; and
generating a medical report based on the at least one content block selected by the user.

2. The medical report generation method according to claim 1, **characterized in that**, further comprising:
receiving a first selection operation for the content block in the analysis display interface; and
in the analysis display interface, presenting related information of the content block in association with the content block selected by the first selection operation, wherein
the related information of the content block is presented in an editable manner.

3. The medical report generation method according to any one of claims 1 to 2, **characterized in that**, generating a medical report based on the at least one content block selected by the user comprises:
acquiring the at least one content block selected by the user; and
presenting content of the content block in a report editing interface in a first preset arrangement manner.

4. The medical report generation method according to claim 2, **characterized in that**, further comprising:
presenting the related information of the content block and the content of the content block in association with each other in the report editing interface.

5. The medical report generation method according to claim 1, **characterized in that**, further comprising:
identifying a display type of an analysis result contained in content of each content block; and
forming area division for a report editing interface according to the identified display type of the analysis result, so that each divided area presents the content of the content block of the corresponding display type respectively.

6. The medical report generation method according to claim 1 or 5, **characterized in that**, further comprising:
identifying a display type of an analysis result contained in the content of each content block, wherein the display type at least comprises one or more of an image type and a table type;
in case that the display type of the analysis result contained in the content of the content block is the table type, acquiring the table content contained in the content; and
generating a corresponding table object according to the table content, and presenting the table object on a report editing interface.

7. The medical report generation method according to claim 3, **characterized in that**, presenting the content of the content block in a report editing interface in a first preset arrangement manner comprises:
presenting a plurality of boxes to be filled in the report editing interface according to the first preset arrangement manner; and
adjusting appearance parameter of the content of the corresponding content block based on display attribute of each box to be filled, so that the content of the content block is presented in a display manner adapted to the box to be filled;
the method further comprising:
based on the report editing interface, filling the content of the content block into a corresponding box to be filled with the adjusted appearance parameter, to generate a medical report containing the selected content block.

8. The medical report generation method according to claim 7, **characterized in that**, further comprising:
matching the first preset arrangement manner in the report editing interface according to the number of the selected content blocks.

9. The medical report generation method according to claim 7, **characterized in that**, further comprising:
upon re-entering the analysis display interface and re-selecting content blocks, matching a corresponding arrangement manner in which the re-selected content blocks are presented on the report editing interface based on the re-selected content blocks.

10. The medical report generation method according to claim 7, **characterized in that**, further comprising:
in case that the number of the selected content blocks exceeds a preset threshold, determining the content of the content blocks to be filled into the boxes to be filled based on an order of selecting the content blocks.

11. The medical report generation method according to any one of claims 1 to 10, **characterized in that**, further comprising:
determining reference information presented in the display area and located outside the content block corresponding to the display area, wherein the reference information is used to assist the user in interpreting the analysis result;
presenting the reference information depending on whether the reference information corresponds to a plurality of analysis results presented on the report editing interface, if so, presenting the reference information without repetition on the report editing interface, in association with the content of content blocks having the analysis results corresponding thereto.

12. The medical report generation method according to claim 11, **characterized in that**, further comprising:
in response to an arrangement operation for adjusting an arrangement position of the content of each content block on the report editing interface,
adjusting an arrangement position of the reference information on the report editing interface based on the arrangement operation.

13. The medical report generation method according to claim 11, **characterized in that**, presenting the reference information in association with the content of content blocks having the analysis results corresponding thereto comprises:
setting the same identification feature for the reference information and the content of the content blocks having the analysis results corresponding thereto.

14. The medical report generation method according to claim 1, **characterized in that**, further comprising:
receiving a second extraction operation by the user with respect to the analysis display interface; and
in response to the second extraction operation, presenting at least one of a plurality of content blocks in a template interface, wherein the content block presented in the template interface is in a selected state.

15. The medical report generation method according to claim 14, **characterized in that**, in response to the second extraction operation, presenting at least one of a plurality of content blocks in a template interface comprises:
determining a first configuration manner for configuring the content blocks on the template interface; and
determining at least part of the plurality of content blocks on the analysis display interface as target content blocks based on the first configuration manner, and determining that the target content blocks are presented on the template interface in a second preset arrangement manner.

16. The medical report generation method according to claim 15, **characterized in that**, further comprising:
receiving an adjustment operation by the user on the first configuration manner on the template interface;
in response to the adjustment operation, generating a second configuration manner corresponding to the adjustment operation; and
presenting a selection box capable of switching the first configuration manner to the second configuration manner on the template interface.

17. The medical report generation method of claim 1, **characterized in that**, after receiving a selection operation by the user with respect to at least one content block, the method further comprises:
generating a third configuration manner corresponding to the selection operation based on the selection operation by the user with respect to the at least one content block, the third configuration manner is used for determining at least part of the plurality of content blocks on the analysis display interface as target content blocks, and determining that the target content blocks are presented on the report editing interface in a third preset arrangement manner.

18. A medical report generation apparatus, **characterized in that**, at least comprising a processor, the processor is configured to perform the medical report generation method of any one of claims 1 to 17.

19. A storage medium storing a computer program, **characterized in that**, the computer program, when executed by a processor, implements the medical report generation method of any one of claims 1 to 17.

20. A functional near-infrared spectroscopy system, **characterized in that**, at least comprising the medical report generation apparatus of claim 18.
